(19) Europäisches Patentamt

European Patent Office

Office européen des brevets

(11) **EP 2 030 609 A2**

(12) **DEMANDE DE BREVET EUROPEEN**

(43) Date de publication:
**04.03.2009 Bulletin 2009/10**

(51) Int Cl.:
**A61K 8/92** *(2006.01)*    **A61Q 1/10** *(2006.01)*

(21) Numéro de dépôt: **08162198.9**

(22) Date de dépôt: **12.08.2008**

(84) Etats contractants désignés:
**AT BE BG CH CY CZ DE DK EE ES FI FR GB GR HR HU IE IS IT LI LT LU LV MC MT NL NO PL PT RO SE SI SK TR**
Etats d'extension désignés:
**AL BA MK RS**

(30) Priorité: **28.08.2007   FR 0757221**
                **28.08.2007   FR 0757220**

(71) Demandeur: **L'Oreal**
**75008 Paris (FR)**

(72) Inventeurs:
 • **Arditty, Stéphane**
  **91160 Balainvilliers (FR)**
 • **Rimbert, Sandrine**
  **92160 Antony (FR)**

(74) Mandataire: **Caillet, Isabelle**
**Bureau Casalonga & Josse**
**Bayerstrasse 71/73**
**80335 München (DE)**

(54) **Composition de soin ou de maquillage comprenant une cire dure et un composé pâteux, procédé de soin ou de maquillage des cils**

(57) L'invention concerne une composition cosmétique comprenant une phase aqueuse, au moins 10 % en poids, par rapport au poids total de la composition, d'au moins une cire dure, et au moins un composé pâteux.

Elle concerne également une composition cosmétique comprenant une phase aqueuse, au moins une cire dure, au moins un composé pâteux, et éventuellement au moins un huile, le rapport pondéral cire(s) dure(s)/(composé(s) pâteux+éventuelle(s) huile(s)) étant compris dans l'intervalle allant de 2 à 8.

Leurs utilisations pour le soin ou le maquillage des fibres kératiniques et notamment des cils sont également décrites.

**EP 2 030 609 A2**

**Description**

L'invention concerne une composition cosmétique comprenant une phase aqueuse, au moins une cire dure et au moins un composé pâteux, un procédé de maquillage ou de soin des fibres kératiniques, comme les cils, les sourcils et les cheveux, et plus particulièrement des cils, mettant en oeuvre ladite composition et son utilisation pour le maquillage desdites fibres, et plus particulièrement comme mascara.

Dans le domaine de la cosmétique, les compositions de revêtement des cils, tels que les mascaras, sont généralement des compositions de maquillage, des compositions à appliquer sur un maquillage (encore appelées « top-coat »), ou encore des compositions de soin cosmétique des cils.

Les mascaras sont notamment préparés selon deux types de formulation : les mascaras aqueux dits mascaras crèmes, sous forme de dispersion de cires dans l'eau, et les mascaras anhydres ou à faible teneur en eau, dits mascaras résistant à l'eau (dits « waterproofs »), sous forme de dispersions de cires dans des solvants organiques.

La présente demande concerne plus spécifiquement le domaine des mascaras aqueux.

L'application de mascara permet d'augmenter le volume des cils et en conséquence d'augmenter l'intensité du regard. Pour cela, il existe de nombreux mascaras épaississants ou chargeants, donnant du volume. Cet effet chargeant est généralement obtenu en déposant un maximum de matières solides sur les cils.

C'est en particulier à travers le choix qualitatif et quantitatif des particules solides, notamment les cires, que peuvent être ajustées les spécificités d'application recherchées pour les compositions de maquillage, comme par exemple leur fluidité ou consistance, leur pouvoir couvrant ainsi que leur pouvoir épaississant (encore appelé pouvoir chargeant ou maquillant).

Afin d'ajuster la consistance et la cosméticité de ces compositions, des cires dites « molles » telles que la cire d'abeille ou la cire de paraffine, sont utilisées en association avec des cires dites « dures », afin d'obtenir un mascara présentant une teneur en solides élevée et une consistance moyenne à élevée, ces caractéristiques étant nécessaires pour qu'un mascara soit chargeant.

En outre, les mascaras aqueux comprennent majoritairement un système tensioactif, par exemple à base de stéarate de triéthanolamine, ce qui permet d'obtenir une dispersion stable de particules de cires agrégées dans une phase aqueuse. Ce système joue un rôle important dans l'obtention d'une telle dispersion, notamment à l'interface dans les interactions entre particules de cire.

Cependant, les mascaras décrits ci-dessus présentent les inconvénients d'avoir un caractère sec à l'application et une consistance qui n'est pas facile à travailler, ainsi que l'obtention d'un dépôt granuleux lorsque la quantité de matières solides est trop élevée.

L'utilisation de cire « molles » dans ces compositions permet d'obtenir des dépôts un peu moins granuleux mais confère au dépôt final un caractère collant, ce qui conduit à un collage des cils maquillés lorsqu'on les frotte simplement avec un doigt.

Il existe donc un besoin de mettre au point une composition cosmétique, notamment de maquillage des cils permettant d'obtenir un un dépôt lisse et homogène sur les cils et un effet chargeant, tout en présentant une consistance facile à travailler après application, et ne séchant pas trop rapidement.

La demanderesse a découvert, de façon surprenante, qu'il était possible de formuler des compositions cosmétiques à phase aqueuse permettant de surmonter les inconvénients présentés ci-dessus, en utilisant dans ces compositions au moins 10 % en poids, par rapport au poids total de la composition, d'au moins une cire dure, et au moins un composé pâteux.

Elle a également trouvé que les inconvénients présentés ci-dessus pouvaient être surmontés avec une composition cosmétique comprenant une phase aqueuse, au moins une cire dure, au moins un composé pâteux, et éventuellement au moins un huile, le rapport pondéral cire(s) dure(s)/(composé(s) pâteux+éventuelle(s) huile(s)) étant compris dans l'intervalle allant de 2 à 8.

L'invention a donc pour objet des compositions cosmétiques, en particulier de maquillage ou de soin des fibres kératiniques, comprenant une phase aqueuse, au moins 10 % en poids, par rapport au poids total de la composition, d'au moins une cire dure, et au moins un composé pâteux.

Elle a aussi pour objet des compositions cosmétiques, en particulier de maquillage ou de soin des fibres kératiniques, comprenant une phase aqueuse, au moins une cire dure, au moins un composé pâteux, et éventuellement au moins un huile, le rapport pondéral cire(s) dure(s)/(composé(s) pâteux+éventuelle(s) huile(s)) étant compris dans l'intervalle allant de 2 à 8.

D'autres objets sont des procédés de maquillage ou de soin des fibres kératiniques, en particulier des cils, des sourcils et des cheveux, et plus particulièrement des cils, mettant en oeuvre les compositions selon l'invention.

Elle concerne également l'utilisation des compositions selon l'invention comme mascaras.

D'autres objets et caractéristiques, aspects et avantages de l'invention apparaîtront encore plus clairement à la lecture de la description et de l'exemple qui suit.

La composition cosmétique selon l'invention comprend une phase aqueuse, au moins 10 % en poids, par rapport au poids total de la composition, d'au moins une cire dure, et au moins un composé pâteux.

Cette composition peut comprend en outre au moins une huile. Le rapport pondéral cire(s) dure(s)/(composé(s) pâteux+éventuelle(s) huile(s)) est alors de préférence compris dans l'intervalle allant de 2 à 8.

Un autre mode de réalisation de l'invention consiste en une composition cosmétique comprenant une phase aqueuse, au moins une cire dure, au moins un composé pâteux, et éventuellement au moins un huile, le rapport pondéral cire(s) dure(s)/(composé(s) pâteux+éventuelle(s) huile(s)) étant compris dans l'intervalle allant de 2 à 8.

Plus préférentiellement, le rapport pondéral cire(s) dure(s)/(composé(s) pâteux+éventuelle(s) huile(s)) est compris dans l'intervalle allant de 2 à 6, mieux encore de 2,5 à 6, et encore plus préférentiellement de 3 à 5.

Les compositions selon l'invention comprennent de préférence une quantité totale de composé(s) pâteux et d'huile(s) supérieure à 1 % en poids, mieux encore allant de 1 à 15 % en poids, préférentiellement supérieure à 2 % en poids, et par exemple allant de 2 à 10 % en poids, et encore plus préférentiellement allant de 3 à 10 % en poids par rapport au poids total de la composition.

La phase aqueuse contenue dans les compositions selon l'invention peut former la phase continue des compositions.

Par composition à phase continue aqueuse, on entend une composition présentant une conductivité, mesurée à 25 °C, supérieure ou égale à 23 $\mu$S/cm (microSiemens/cm), la conductivité étant mesurée, par exemple, à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

La phase aqueuse comprend de l'eau et éventuellement au moins un solvant hydrosoluble.

Par "solvant hydrosoluble", on entend au sens de la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent en outre être volatils.

Parmi les solvants hydrosolubles pouvant être utilisés dans les compositions conformes à l'invention, on peut citer notamment les monoalcools inférieurs ayant de 1 à 5 atomes de carbone tels que l'éthanol et l'isopropanol, les alkylène glycols ayant de 2 à 8 atomes de carbone tels que l'éthylène glycol, le propylène glycol, le 1,3-butylène glycol et le dipropylène glycol, les cétones en $C_3$-$C_4$ et les aldéhydes en $C_2$-$C_4$.

La phase aqueuse est généralement présente dans les compositions selon l'invention en une teneur allant de 1 à 95 % en poids, par rapport au poids total de la composition, de préférence allant de 3 à 80 % en poids, et préférentiellement allant de 5 à 60 % en poids.

Par cire au sens de la présente invention, on entend un composé lipophile, solide à température ambiante (25 °C), à changement d'état solide/liquide réversible, ayant une température de fusion supérieure ou égale à 30°C, et pouvant aller jusqu'à 120°C. En particulier, les cires présentent une température de fusion supérieure à 30°C et mieux supérieure à 45°C.

Au sens de l'invention, la température de fusion correspond à la température du pic le plus endothermique observé en analyse thermique (analyse calorimétrique différentielle ou DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Par cire dure, on entend au sens de la présente invention, une cire présentant à 20 °C, une dureté supérieure à 5 MPa, notamment allant de 5 à 30 MPa, de préférence supérieure à 6 MPa, mieux encore allant de 6 à 25 MPa.

La dureté de la cire est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant : on fait fondre la cire à une température égale à la température de fusion de la cire + 10 °C. On fait couler la cire fondue dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.

Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.

La valeur de la dureté est la force de compression maximale mesurée divisée par la surface du cylindre du texturomètre en contact avec la cire.

Les cires peuvent être hydrocarbonées, fluorées et/ou siliconées, et être d'origine végétale, minérale, animale et/ou synthétique.

A titre d'exemples de cire dure, on peut notamment citer la cire de Carnauba, la cire de candelilla, la cire BIS-PEG-12 DIMETHICONE CANDELILLATE comme, par exemple, la Siliconyl Candellila Wax commercialisée par la société KOSTER KEUNEN, la cire de Jojoba hydrogénée telle que, par exemple, celle commercialisée par la société DESERT WHALE, l'huile de palme hydrogénée telle que celle commercialisée par la société SIO, la cire de son de riz, la cire de Sumac, les cires de cérésine, la cire de laurier, la cire d'insecte Chinois, la cire de Shellac, l'huile d'olive hydrogénée telle que la Waxolive de la société SOLIANCE, les cires obtenues par hydrogénation d'huile d'olive estérifiée avec des alcool gras à chaîne en C12 à C18 telle que celles vendues par la société SOPHIM sous les dénominations commerciales Phytowax Olive 12L44, 14L48, 16L55 et 18L57, les cires obtenues par hydrogénation d'huile de ricin estérifiée avec l'alcool cétylique ou béhénique comme par exemple celle qui sont vendues sous les dénominations Phytowax Ricin 16 L 64 et Phytowax Ricin 22 L 73 par la société SOPHIM, la cire de Cameline hydrogénée, la cire d'Ouricury, la cire de Montan, les cires d'ozokerite comme, par exemple, la Wax SP 1020 P commercialisée par la société Strahl & Pitsch, les cires microcristallines comme, par exemple, celle vendue sous la dénomination commerciale Microwax HW par la société PARAMELT, les triglycérides d'acides laurique, palmitique, cétylique et stéarique (nom INCI : coco-glycérides hydrogénés) tel que, par exemple, celui vendu sous la dénomination commerciale Softisan 100 par la société SASOL, les cires de polyméthylène comme, par exemple, celle vendue sous la dénomination commerciale Cirebelle 303 par la société SASOL, les cires de polyéthylène comme, par exemple, celles vendues sous les dénominations commerciales Performalene 400 polyethylene, Performalene 655 polyethylene et Performalene 500-L polyethylene par la société New Phase Technologies, les cires d'alcool-polyéthylène comme, par exemple, celle commercialisée sous le nom Performacol 425 Alcohol par la société BARECO, le copolymère éthylène/acide acrylique 95/5 vendu sous la dénomination commerciale cire AC 540 par la société Honeywell, l'hydroxystéarate d'hydroxyoctacosanyle comme, par exemple, celui vendu sous la dénomination commerciale l'Elfacos C 26 par la société AKZO, le stéarate d'octacosanyle comme, par exemple, celui commercialisé sous la dénomination Kester Wax K 82 H par la société KOSTER KEUNEN, le stéarate de stéaryle comme, par exemple, celui commercialisé sous la dénomination Liponate SS par la société LIPO CHEMICALS, le distéarate de pentaérythritol comme, par exemple, celui commercialisé sous la dénomination Cutina PES par la société COGNIS, le mélange d'adipate de dibéhényle, d'adipate de dioctadécyle et d'adipate de di-eicosanyle (nom INCI adipate de dialkyle en $C_{18-22}$), le mélange d'adipate de dilauryle et d'adipate de ditétradécyle (nom INCI : adipate de dialkyle en $C_{12-14}$), le mélange de sébaçate de dioctadécyle, de sébaçate didocosyle et de sébaçate dieicosyle (nom INCI : sébacate de dialkyle en $C_{18-22}$), le mélange d'octadécanedioate de dioctadécyle, d'octanedioate de didocosyle et d'octanedioate de dieicosyle (nom INCI : octanedioate de dialkyle en $C_{18-22}$) comme, par exemple, ceux commercialisés par la société COGNIS, le tétrastéarate de pentaérythrityle comme, par exemple, le Liponate PS-4 de la société Lipo Chemicals, le stéarate de tétracontanyle comme, par exemple, la Kester Wax K76 H de la société KOSTER KEUNEN, le benzoate de stéaryle comme, par exemple, le Finsolv 116 de la société FINETEX, le fumarate de béhényle comme, par exemple, le Marrix 222 de la société AKZO BERNEL, le tétrastéarate de di-(triméthylol-1,1,1-propane) comme, par exemple, celui qui est proposé sous la dénomination « HEST 2T-4S » par la société HETERENE, le distéarate de didotriacontanyle comme, par exemple, la Kester Wax K82D de la société KOSTER KEUNEN, le montanate de polyéthylène glycol à 4 motifs oxyéthylène (PEG-4) comme, par exemple, celui qui est vendu sous la dénomination commerciale Clariant Licowax KST1, le disalycilate d'hexanediol comme, par exemple, la Betawax RX-13750 commercialisée par la société CP Hall, l'hexastéarate de dipentaérythrytol comme, par exemple, celui qui est vendu sous la dénomination commerciale Hest 2P-6S par la société HETERENE, le tétrabéhénate de ditriméthylolpropane comme, par exemple, celui qui est vendu sous la dénomination commerciale Hest 2T-4B par la société HETERENE, les esters de Jojoba comme, par exemple, celui qui est vendu sous la dénomination commerciale Floraester HIP par la société FLORATECH, les mélanges d'acide carboxylique linéaire (C20-40)/hydrocarbures saturés (nom INCI : C20-40 acid polyethylene) comme, par exemple, Performacid 350 acid de la société NEW PHASE TECHNOLOGIES, la cire synthétique de type Fischer-Tropsch telle que celle commercialisée sous la référence Rosswax 100 par la société ROSS, l'alcool cétylique, l'alcool stéarique, l'alcool béhénique, le carbonate de dioctadécyle comme, par exemple, Cutina KE 3737, le polybéhénate de saccharose comme, par exemple, le Crodaderm B de la société CRODA, et leurs mélanges.

On peut également utiliser les cires citées ci-dessus sous la forme de mélanges disponibles dans le commerce, par exemple, sous les dénominations KOSTER KPC-56 (Mélange de 87,5 % en poids de stéarate de cétyle, de 7,5 % en poids d'alcool béhénique et de 5 % en poids de glycérides palm kernel), KPC-60 (Mélange de 87,5 % en poids de stéarate de stéaryle, de 7,5 % en poids d'alcool béhénique et de 5 % en poids de glycérides palm kernel), KPC-63 (Mélange de 87,5 % en poids de stéarate de béhényle, de 7,5 % en poids d'alcool béhénique et de 5 % en poids de glycérides palm kernel) et KPC-80 (Mélange de 86 % en poids de cire d'abeille synthétique, de 7,5 % d'huile végétale hydrogénée et de 6,5 % en poids d'alcool béhénique) de la société KOSTER KEUNEN.

On utilise de préférence des cires d'origine végétale telles que la cire de carnauba, la cire de candellila, la cire de jojoba hydrogénée, la cire de sumac, les cires obtenues par hydrogénation d'huile d'olive estérifiée avec les alcool gras à chaîne en C12 à C18 vendues par la société SOPHIM dans la gamme Phytowax (12L44, 14L48, 16L55 et 18L57), la cire de son de riz, les alcools cétylique, stéarylique et béhéniques, la cire de laurier, la cire d'Ouricury.

La ou les cire(s) dure(s) sont présentes de préférence en une quantité d'au moins 12 % en poids, mieux encore de 12 à 30 % en poids, encore plus préférentiellement d'au moins 14 % en poids, et surtout de 14 à 25 % en poids par rapport au poids total de la composition.

Par "composé pâteux" au sens de la présente invention, on entend un composé gras lipophile à changement d'état solide/liquide réversible et comportant à la température de 23°C une fraction liquide et une fraction solide.

Un composé pâteux est à la température de 23°C, sous la forme d'une fraction liquide et d'une fraction solide. En d'autres termes, la température de fusion commençante du composé pâteux est inférieure à 23°C. La fraction liquide du composé pâteux, mesurée à 23°C, représente de 20 à 97% en poids du composé pâteux. Cette fraction liquide à 23°C représente plus préférentiellement de 25 à 85%, et mieux de 30 à 60% en poids du composé pâteux.

La fraction liquide en poids du composé pâteux à 23°C est égale au rapport de l'enthalpie de fusion consommée à 23°C sur l'enthalpie de fusion du composé pâteux.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

La fraction liquide du composé pâteux, mesurée à 32°C, représente de préférence de 40 à 100% en poids du composé pâteux, mieux encore de 50 à 100% en poids du composé pâteux. Lorsque la fraction liquide du composé pâteux mesurée à 32°C est égale à 100%, la température de la fin de la plage de fusion du composé pâteux est inférieure ou égale à 32°C.

La fraction liquide du composé pâteux, mesurée à 32°C, est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux a de préférence une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon, la profondeur de pénétration étant de 0,3 mm. La valeur relevée de la dureté est celle du pic maximum.

Le composé pâteux peut être choisi parmi les composés synthétiques et les composés d'origine végétale. Un composé pâteux peut être obtenu par synthèse à partir de produits de départ d'origine végétale.

Le composé pâteux est avantageusement choisi parmi :

- la lanoline et ses dérivés tels que l'alcool de lanoline, les lanolines oxyéthylénées, la lanoline acétylée, les esters de lanoline tels que le lanolate d'isopropyle, les lanolines oxypropylénées,
- les composés siliconés polymères ou non-polymères comme les polydiméthysiloxanes de masses moléculaires élevées, les polydiméthysiloxanes à chaînes latérales du type alkyle ou alcoxy ayant de 8 à 24 atomes de carbone, notamment les stéaryl diméthicones,
- les composés fluorés polymères ou non-polymères,
- les polymères vinyliques, notamment

  • les homopolymères d'oléfines,
  • les copolymères d'oléfines,
  • les homopolymères et copolymères de diènes hydrogénés,
  • les oligomères linéaires ou ramifiés, homo ou copolymères de (méth)acrylates d'alkyle ayant de préférence un

groupement alkyle en $C_8$-$C_{30}$
- les oligomères homo et copolymères d'esters vinyliques ayant des groupements alkyles en $C_8$-$C_{30}$,
- les oligomères homo et copolymères de vinyléthers ayant des groupements alkyles en $C_8$-$C_{30}$,

- les polyéthers liposolubles résultant de la polyéthérification entre un ou plusieurs diols en $C_2$-$C_{100}$, de préférence en $C_2$-$C_{50}$,
- les esters et les polyesters,
- et leurs mélanges.

Le composé pâteux peut être un polymère, notamment hydrocarboné.

Un composé pâteux siliconé et fluoré préféré est le polyméthyl-trifluoropropyl-méthylalkyl-diméthylsiloxane, fabriqué sous la dénomination X22-1088 par SHIN ETSU.

Lorsque le composé pâteux est un polymère siliconé et/ou fluoré, la composition comprend avantageusement un agent compatibilisant tel que les esters à courte chaîne comme le néopentanoate d'isodécyle.

Parmi les polyéthers liposolubles, on peut notamment citer les copolymères d'oxyde d'éthylène et/ou d'oxyde de propylène avec des oxydes d'alkylène en $C_6$-$C_{30}$. De préférence, le rapport pondéral de l'oxyde d'éthylène et/ou de l'oxyde de propylène avec les oxydes d'alkylène dans le copolymère est de 5:95 à 70:30. Dans cette famille, on citera notamment les copolymères blocs comprenant des blocs d'oxydes d'alkylène en $C_6$-$C_{30}$ ayant un poids moléculaire allant de 1 000 à 10 000, par exemple un copolymère bloc polyoxyéthylène/polydodécylène glycol tel que les éthers de dodécanediol (22 mol) et de polyéthylène glycol (45 motifs oxyéthylène ou OE) commercialisés sous la marque ELFACOS ST9 par Akzo Nobel.

Parmi les esters, on préfère notamment :

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras comme l'acide stéarique, l'acide caprique, l'acide stéarique, l'acide isostéarique et l'acide 12-hydroxystéarique, tels que ceux notamment commercialisés sous la marque Softisan 649 par la société Sasol ;
- les esters de phytostérol ;
- les esters de pentaérythritol ;
- les esters formés à partir :

  - d'au moins un alcool en $C_{16-40}$, l'un au moins des alcools étant un alcool de Guerbet et
  - d'un dimère diacide formé à partir d'au moins un acide gras insaturé en C18-40,

  comme l'ester de dimère d'acides gras de tallol comprenant 36 atomes de carbone et d'un mélange i) d'alcools de Guerbet comprenant 32 atomes de carbone et ii) d'alcool béhénylique ; l'ester de dimère d'acide linoléique et d'un mélange de deux alcools de Guerbet, le 2-tétradécyl-octadécanol (32 atomes de carbone) et le 2-hexadécyl-eicosanol (36 atomes de carbone) ;
- les polyesters non réticulés résultant de la polycondensation entre un acide dicarboxylique ou un acide polycarboxylique, linéaire ou ramifié, en $C_4$-$C_{50}$, et un diol ou un polyol en $C_2$-$C_{50}$ ;
- les polyesters qui résultent de l'estérification entre un acide polycarboxylique et un ester d'acide carboxylique hydroxylé aliphatique comme le Risocast DA-L et le Risocast DA-H commercialisés par la société japonaise KOKYU ALCOHOL KOGYO, qui sont des esters résultant de la réaction d'estérification de l'huile de ricin hydrogénée avec l'acide dilinoléïque ou l'acide isostéarique ; et
- les esters aliphatiques d'ester résultant de l'estérification entre un ester d'acide carboxylique hydroxylé aliphatique et un acide carboxylique aliphatique, par exemple celui vendu sous la dénomination commerciale Salacos HCIS (V)-L par la société Nishing Oil.

Un alcool de Guerbet est le produit réactionnel de la réaction de Guerbet bien connue de l'homme du métier. Il s'agit d'une réaction transformant un alcool aliphatique primaire en son alcool dimère β-alkylé avec perte d'un équivalent d'eau.

Les acides carboxyliques aliphatiques décrits ci-dessus comprennent généralement de 4 à 30 et de préférence de 8 à 30 atomes de carbone. Ils sont choisis de préférence parmi l'acide héxanoïque, l'acide heptanoïque, l'acide octanoïque, l'acide 2-éthylhexanoïque, l'acide nonanoïque, l'acide décanoïque, l'acide undécanoïque, acide dodécanoïque, l'acide tridécanoïque, l'acide tétradécanoïque, l'acide pentadécanoïque, l'acide hexadécanoïque, l'acide hexyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

Les acides carboxyliques aliphatiques sont de préférence ramifiés.

Les esters d'acide carboxylique aliphatique hydroxylé sont avantageusement issus d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. Les esters d'acide carboxylique aliphatique hydroxylé sont notamment choisis parmi :

a) les esters, partiels ou totaux, d'acides monocarboxyliques aliphatiques monohydroxylés linéaires, saturés ;
b) les esters, partiels ou totaux, d'acides monocarboxyliques aliphatiques monohydroxylés insaturés ;
c) les esters, partiels ou totaux, de polyacides carboxyliques aliphatiques monohydroxylés saturés ;
d) les esters, partiels ou totaux, de polyacides carboxyliques aliphatiques polyhydroxylés saturés ;
e) les esters, partiels ou totaux, de polyols aliphatiques en $C_2$ à $C_{16}$ ayant réagi avec un acide mono- ou un polycarboxylique aliphatique mono- ou polyhydroxylé,
f) et leurs mélanges.

Les esters aliphatiques d'ester sont avantageusement choisis parmi :

- l'ester résultant de la réaction d'estérification de l'huile de ricin hydrogénée avec l'acide isostéarique dans les proportions 1 pour 1 (1/1), qui est appelé monoisostéarate d'huile de ricin hydrogénée,
- l'ester résultant de la réaction d'estérification de l'huile de ricin hydrogénée avec l'acide isostéarique dans les proportions 1 pour 2 (1/2), qui est appelé le diisostéarate d'huile de ricin hydrogénée,
- l'ester résultant de la réaction d'estérification de l'huile de ricin hydrogénée avec l'acide isostéarique dans les proportions 1 pour 3 (1/3), qui est appelé le triisostéarate d'huile de ricin hydrogénée,
- et leurs mélanges.

De préférence, le composé pâteux est choisi parmi les composés d'origine végétale.

Parmi ceux-ci, on peut notamment citer l'huile de jojoba isomérisée telle que l'huile de jojoba partiellement hydrogénée isomérisée trans fabriquée ou commercialisée par la société Desert Whale sous la référence commerciale Iso-Jojoba-50®, la cire d'orange comme, par exemple, celle qui est commercalisée sous la référence Orange Peel Wax par la société Koster Keunen, le beurre de karité, l'huile d'olive partiellement hydrogénée comme, par exemple, le composé commercialisé sous la référence Beurrolive par la société Soliance, le beurre de cacao, l'huile de mangue comme, par exemple, la Lipex 302 de la société Aarhuskarlshamn.

Le ou les composés pâteux sont présents de préférence en une quantité supérieure ou égale à 1% en poids par rapport au poids total de la composition, par exemple de 1 à 15% en poids, mieux en une quantité supérieure ou égale à 2% en poids, allant par exemple de 2 à 10 % en poids, et encore plus préférentiellement de 3 à 8 % en poids, par rapport au poids total de la composition.

Les compositions selon l'invention présentent avantageusement une teneur en matière sèche (ou extrait sec) supérieure ou égale à 40% en poids, mieux supérieure ou égale à 45% en poids par rapport au poids total de la composition.

La teneur en matière sèche, c'est à dire la teneur en matière non volatile, peut être mesurée de différentes manières. On peut citer par exemple les méthodes par séchage à l'étuve, les méthodes par séchage par exposition à un rayonnement infrarouge ainsi que les méthodes chimiques par titrage de l'eau selon Karl Fischer.

De préférence, la quantité de matière sèche, communément appelée « extrait sec », des compositions selon l'invention, est mesurée par échauffement de l'échantillon par des rayons infrarouges de 2 $\mu$m à 3,5 $\mu$m de longueur d'onde. Les substances contenues dans lesdites compositions qui possèdent une pression de vapeur élevée, s'évaporent sous l'effet de ce rayonnement. La mesure de la perte de poids de l'échantillon permet de déterminer « l'extrait sec » de la composition. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant : on étale environ 1g de la composition sur une coupelle métallique. Celle-ci, après introduction dans le dessiccateur, est soumise à une consigne de température de 120 °C pendant une heure. La masse humide de l'échantillon correspondant à la masse initiale, et la masse sèche de l'échantillon correspondant à la masse après exposition au rayonnement, sont mesurées au moyen d'une balance de précision.

La teneur en matière sèche est calculée de la manière suivante :

$$\text{Extrait Sec} = 100 \text{ x (masse sèche / masse humide).}$$

Les compositions selon l'invention peuvent comprendre en outre au moins une huile.

Par « huile », on entend un corps non aqueux liquide à température ambiante et pression atmosphérique. Elle présente généralement une température de fusion inférieure à 55 °C, de préférence inférieure à 50 °C, et mieux encore inférieure ou égale à 45 °C. L'huile peut être volatile ou non volatile.

Par « huile volatile », on entend au sens de l'invention tout milieu non aqueux susceptible de s'évaporer au contact des fibres kératiniques en moins d'une heure, à température ambiante et pression atmosphérique. Les huiles volatiles pouvant être utilisées dans les compositions de l'invention sont des huiles cosmétiques volatiles, liquides à température ambiante, ayant une pression de vapeur non nulle, à température ambiante et pression atmosphérique, allant de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par « huile non volatile », on entend une huile restant sur les fibres kératiniques à température ambiante et pression atmosphérique au moins plusieurs heures et ayant notamment une pression de vapeur inférieure à $10^{-3}$ mm de Hg (0,13Pa).

La composition selon l'invention peut comprendre des huiles volatiles et/ou des huiles non volatiles, ou leurs mélanges. Elles peuvent être des huiles hydrocarbonées, des huiles siliconées, des huiles fluorées ou leurs mélanges.

On entend par « huile hydrocarbonée », une huile contenant principalement des atomes d'hydrogène et de carbone, et éventuellement des atomes d'oxygène, d'azote, de soufre, de phosphore.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux "d'Isopars®" ou de "Permetyls®" ; les esters ramifiés en $C_8$-$C_{16}$ tels que le néopentanoate d'iso-hexyle ; et leurs mélanges. D'autres huiles hydrocarbonées volatiles comme les distillats de pétrole, notamment ceux vendus sous la dénomination "Shell Solt®" par la société SHELL, peuvent aussi être utilisées.

Comme huiles volatiles, on peut aussi utiliser les silicones volatiles comme, par exemple, les huiles de silicone, linéaire ou cyclique, volatiles, notamment celles ayant une viscosité $\leq$ 6 centistokes ($6.10^{-6}$ m$^2$/s), et ayant notamment de 3 à 6 atomes de silicium, ces silicones comportant éventuellement un ou plusieurs groupes alkyle ou alcoxy ayant 1 ou 2 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser comme huile volatile, des solvants organiques volatils notamment fluorés tels que le nonafluorométhoxy butane ou le perfluorométhylcyclopentane.

Comme huile hydrocarbonée non volatile, on peut notamment citer :

- les huiles hydrocarbonées d'origine végétale, telles que les triglycérides constitués d'esters d'acides gras linéaires ou ramifiés, saturés ou insaturés, en $C_4$-$C_{24}$ et de glycérol. A titre d'exemples de telles huiles, on peut notamment citer les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore et de rosier muscat ; ou encore les triglycérides des acides caprylique/caprique comme ceux vendus par la société Stéarineries Dubois ou ceux vendus sous les dénominations de Miglyol 810®, 812® et 818® par la société Dynamit Nobel ;
- les éthers de synthèse ayant de 10 à 40 atomes de carbone ;
- les hydrocarbures linéaires ou ramifiés, d'origine minérale ou synthétique, tels que la vaseline, les polydécènes, le polyisobutène hydrogéné tel que le parléam, le squalane, et leurs mélanges;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone, à condition que $R_1 + R_2$ soit $\geq$ 10. A titre d'exemples d'ester de synthèse, on peut notamment citer l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthylhexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras saturés ou insaturés, en $C_{12-24}$ tels que l'acide oléique, l'acide linoléique et l'acide linolénique ;
- et leurs mélanges.

Les huiles de silicone non volatiles utilisables dans les compositions conformes à l'invention peuvent être les polydiméthylsiloxanes (PDMS) non volatiles, les polydiméthylsiloxanes comportant des groupements alkyle ou alcoxy, latéraux et/ou en bout de chaîne siliconée, groupements comportant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes et les 2-phényléthyl trimethylsiloxysilicates ;

Les huiles fluorées non volatiles utilisables dans les compositions conformes à l'invention sont notamment des huiles fluorosiliconées, des polyéthers fluorés, des silicones fluorées telles que celles décrites dans le document EP-A-847752.

Lorsqu'elle(s) est (sont) présente(s) dans la composition selon l'invention, la teneur en huile(s) dans la composition conforme à l'invention va de 0,01 à 30 % en poids, en particulier de 0,1 à 25 % en poids, et mieux de 0,1 à 20 % par rapport au poids total de la composition.

Les compositions peuvent comprendre en outre au moins une cire additionnelle présentant une dureté inférieure ou égale à 5 MPa, plus particulièrement allant par exemple de 0,01 à 5 MPa, à 20 °C. La dureté de la cire additionnelle est mesurée de la même manière que pour la cire dure.

La cire additionnelle peut être choisie parmi, par exemple, les cires d'abeille, la cire d'abeille siliconée, les cires de paraffine, la cire de berry, la cire de citron, le copolymère éthylène/acétate de vinyle 85/15 vendu sous la dénomination commerciale Cire AC 400, et les cires de silicone telles que l'alkyldimethicone vendue sous la dénomination commerciale SF 1642 par la société GE Bayer silicone, les cires vendues sous les dénominations commerciales K82P-S et K82P-VS par la société Koster Keunen.

On peut aussi citer des cires obtenues par hydrogénation catalytique d'huiles animales ou végétales ayant des chaînes grasses, linéaires ou ramifiées, en $C_8$-$C_{32}$ comme l'huile de tournesol hydrogénée, l'huile de ricin hydrogénée, l'huile de coprah hydrogénée et l'huile de lanoline hydrogénée.

On peut encore citer les cires de silicone et les cires fluorées.

La cire additionnelle peut être une cire dite cire collante, c'est-à-dire présentant un collant supérieur ou égal à 0,1 N.s et une dureté inférieure ou égale à 3,5 MPa.

La cire collante utilisée peut présenter notamment un collant allant de 0,1 N.s à 10 N.s, en, particulier allant de 0,1 N.s à 5 N.s, de préférence allant de 0,2 à 5 N.s et mieux allant de 0,3 à 2 N.s.

Le collant de la cire est déterminé par la mesure de l'évolution de la force de compression en fonction du temps, à 20 °C selon le protocole indiqué précédemment pour la mesure de la dureté.

Pendant le temps de relaxation de 1 s, la force de compression décroît fortement jusqu'à devenir nulle puis, lors du retrait du mobile, la force d'étirement devient négative pour ensuite croître à nouveau vers la valeur 0. Le collant correspond à l'intégrale de la courbe de la force en fonction du temps pour la partie de la courbe correspondant aux valeurs négatives de la force. La valeur du collant est exprimée en N.s.

La cire collante pouvant être utilisée a généralement une dureté inférieure ou égale à 3,5 MPa, en particulier allant de 0,01 à 3,5 MPa, notamment allant de 0,05 à 3 MPa.

Comme cire collante, on peut utiliser un (hydroxystéaroyl)stéarate d'alkyle en $C_{18}$-$C_{38}$ (le groupe alkyle comprenant de 18 à 38 atomes de carbone), seul ou en mélange.

Une telle cire est notamment vendue sous les dénominations « Kester Wax K 82 P® », « Hydroxypolyester K 82 P® » et « Kester Wax K 80 P® » par la société KOSTER KEUNEN.

Dans la présente invention, on peut également utiliser des cires fournies sous forme de petites particules ayant une dimension exprimée en diamètre « effectif » moyen en volume D[4,3] de l'ordre de 0,5 à 30 micromètres, en particulier de 1 à 20 micromètres, et plus particulièrement de 5 à 10 micromètres, désignées par la suite par l'expression « micro cires ».

Les tailles de particules peuvent être mesurées par différentes techniques, telles que les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles des particules des compositions selon l'invention, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

La composition est caractérisée par son diamètre "effectif" moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.

Les mesures sont réalisées à 25 °C, sur une dispersion de particules diluée, obtenue à partir de la composition de la manière suivante : 1) dilution d'un facteur 100 avec de l'eau, 2) homogénéisation de la solution, 3) repos de la solution durant 18 heures, 4) récupération du surnageant homogène blanchâtre.

Le diamètre « effectif » est obtenu en prenant un indice de réfraction de 1,33 pour l'eau et un indice de réfraction moyen de 1,42 pour les particules.

Lorsqu'elle(s) est (sont) présente(s) dans la composition selon l'invention, la teneur en cire(s) additionelle(s) est comprise dans l'intervalle allant de 0,5 à 15 % en poids, mieux encore de 1 à 10 % en poids par rapport au poids total de la composition.

Les compositions selon l'invention peuvent contenir un ou plusieurs agents tensioactifs émulsionnants présents notamment en une quantité allant de 0,1 à 20 %, et mieux de 0,3 % à 15 % en poids par rapport au poids total de la composition.

Selon l'invention, on utilise généralement un agent tensioactif émulsionnant choisi de manière appropriée pour l'obtention d'une émulsion huile-dans-eau. En particulier, on peut utiliser un agent tensioactif émulsionnant possédant à 25 °C une balance HLB (hydrophile-lipophile balance) au sens de GRIFFIN, supérieure ou égale à 8.

La valeur HLB selon GRIFFIN est définie dans J. Soc. Cosm. Chem. 1954 (volume 5), pages 249-256.

Ces agents tensioactifs peuvent être choisis parmi des agents tensioactifs non ioniques, anioniques, cationiques, amphotères. On peut se reporter au document « Encyclopedia of Chemical Technology, KIRK-OTHMER », volume 22, p. 333-432, 3ème édition, 1979, WILEY, pour la définition des propriétés et des fonctions émulsionnantes des agents tensioactifs, en particulier p. 347-377 de cette référence, pour les agents tensioactifs anioniques, amphotères et non ioniques.

Les agents tensioactifs utilisés préférentiellement dans la composition selon l'invention sont choisis parmi :

a) les agents tensioactifs non ioniques de HLB supérieur ou égal à 8 à 25 °C, utilisés seuls ou en mélange; on peut citer notamment :

les éthers de glycérol, oxyéthylénés et/ou oxypropylénés, pouvant comporter de 1 à 150 motifs oxyéthylène et/ou oxypropylène ;

les éthers oxyéthylénés et/ou oxypropylénés (pouvant comporter de 1 à 150 motifs oxyéthylène et/ou oxypropylène) d'alcools gras, notamment en $C_8$-$C_{24}$, et de préférence en $C_{12}$-$C_{18}$, tels que l'éther oxyéthyléné de l'alcool stéarylique à 20 motifs oxyéthylène (nom CTFA "Steareth-20 ") comme le BRIJ 78 commercialisé par la société UNIQEMA, l'éther oxyéthyléné de l'alcool cétéarylique à 30 motifs oxyéthylène (nom CTFA "Ceteareth-30 ") et l'éther oxyéthyléné du mélange d'alcools gras en $C_{12}$-$C_{15}$ comportant 7 motifs oxyéthylène (nom CTFA "$C_{12-15}$ Pareth-7") comme celui commercialisé sous la dénomination NEODOL 25-7® par SHELL CHEMICALS ;

les esters d'acide gras, notamment en $C_8$-$C_{24}$, et de préférence en $C_{16}$-$C_{22}$, et de polyéthylène glycol (ou PEG) (pouvant comprendre de 1 à 150 motifs oxyéthylène), tels que le stéarate de PEG-50 et le monostéarate de PEG-40 commercialisé sous le nom MYRJ 52P® par la société UNIQEMA ;

les esters d'acide gras, notamment en $C_8$-$C_{24}$, et de préférence en $C_{16}$-$C_{22}$, et d'éthers de glycérol oxyéthylénés et/ou oxypropylénés (pouvant comporter de 1 à 150 motifs oxyéthylène et/ou oxypropylène), comme le monostéarate de glycéryle polyoxyéthyléné à 200 motifs oxyéthylène, vendu sous la dénomination Simulsol 220 TM® par la société SEPPIC ; le stéarate de glycéryle polyoxyéthyléné à 30 motifs oxyéthylène comme le produit TAGAT S® vendu par la société GOLDSCHMIDT, l'oléate de glycéryle polyoxyéthyléné à 30 motifs oxyéthylène comme le produit TAGAT O® vendu par la société GOLDSCHMIDT, le cocoate de glycéryle polyoxyéthyléné à 30 motifs oxyéthylène comme le produit VARIONIC LI 13® vendu par la société SHEREX, l'isostéarate de glycéryle polyoxyéthyléné à 30 motifs oxyéthylène comme le produit TAGAT L® vendu par la société GOLDSCHMIDT et le laurate de glycéryle polyoxyéthyléné à 30 motifs oxyéthylène comme le produit TAGAT I® de la société GOLDSCHMIDT ;

les esters d'acide gras, notamment en $C_8$-$C_{24}$, et de préférence en $C_{16}$-$C_{22}$, et d'éthers de sorbitol oxyéthylénés et/ou oxypropylénés (pouvant comporter de 1 à 150 motifs oxyéthylène et/ou oxypropylène), comme le polysorbate 60 vendu sous la dénomination Tween 60® par la société UNIQEMA ;

le diméthicone copolyol, tel que celui vendu sous la dénomination Q2-5220® par la société DOW CORNING ;

le diméthicone copolyol benzoate tel que celui vendu sous la dénomination FINSOLV SLB 101® et 201® par la société FINTEX ;
les copolymères d'oxyde de propylène et d'oxyde d'éthylène, également appelés polycondensats OE/OP, et leurs mélanges.

Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

formule dans laquelle a va de 2 à 120, et b va de 1 à 100.

Les polycondensats OE/OP ont de préférence une masse moléculaire moyenne en poids allant de 1 000 à 15 000, et mieux allant de 2 000 à 13 000. Avantageusement, lesdits polycondensats OE/OP ont une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol/polypropylène glycol/polyéthylène glycol vendus sous les dénominations SYNPERONIC® comme les SYNPERONIC PE/ L44® et SYNPERONIC PE/F127® par la société ICI.

b) les agents tensioactif non ioniques de HLB inférieur à 8 à 25 °C, éventuellement associés à un ou plusieurs agents tensioactif non ioniques de HLB supérieur à 8 à 25 °C, tels que cités ci-dessus, comme :

les esters et éthers d'oses tels que les stéarate de saccharose, cocoate de saccharose, stéarate de sorbitan et leurs mélanges, par exemple l'Arlatone 2121® commercialisé par la société ICI ou le SPAN 65V de la société UNIQEMA ;
les esters d'acides gras, notamment en $C_8$-$C_{24}$, et de préférence en $C_{16}$-$C_{22}$, et de polyol, notamment de glycérol ou de sorbitol, tels que le stéarate de glycéryle, par exemple vendu sous la dénomination TEGIN M® par la société GOLDSCHMIDT, le laurate de glycéryle tel que le produit vendu sous la dénomination IMWITOR 312® par la société HULS, le stéarate de polyglycéryle-2, le tristéarate de sorbitan, et le ricinoléate de glycéryle ;
les éthers oxyéthylénés et/ou oxypropylénés tels que l'éther oxyéthyléné de l'alcool stéarylique à 2 motifs oxyéthylène (nom CTFA "Steareth-2 ") tel que le BRIJ 72 commercialisé par la société UNIQEMA ;
le mélange de cyclométhicone/diméthicone copolyol vendu sous la dénomination Q2-3225C® par la société DOW CORNING ;

c) les tensioactifs anioniques tels que :

les sels d'acides gras en $C_{16}$-$C_{30}$, notamment les sels aminés comme le stéarate de triéthanolamine ou le stéarate de 2-amino-2-méthylpropane-1,3-diol ;
les sels d'acides gras polyoxyéthylénés, notamment les sels aminés ou les sels de métaux alcalins, et leurs mélanges ;
les esters phosphoriques et leurs sels tels que le "DEA oleth-10 phosphate" (Crodafos N 10N de la société CRODA) ou le phosphate de monocétyle monopotassique (Amphisol K de Givaudan ou ARLATONE MAP 160K de la société UNIQEMA) ;
les sulfosuccinates tels que le "Disodium PEG-5 citrate lauryl sulfosuccinate" et le "Disodium ricinoleamido MEA sulfosuccinate" ;
les alkyléthersulfates tels que le lauryl éther sulfate de sodium ;
les iséthionates ;
les acylglutamates tels que le "Disodium hydrogenated tallow glutamate" (AMISOFT HS-21 R® commercialisé par la société AJINOMOTO) et leurs mélanges.

A titre d'exemples de tensioactif cationique, on peut notamment citer :

- les alkylimidazolidiniums tels que l'étho-sulfate d'isostéaryl-éthylimidonium,
- les sels d'ammonium tels que les halogénures d'(alkyl en $C_{12\text{-}30}$)-tri(alkyl en $C_{1\text{-}4}$)ammonium comme le chlorure de N,N,N-triméthyl-1-docosanaminium (ou chlorure de Behentrimonium).

Les compositions selon l'invention peuvent également contenir un ou plusieurs tensioactifs amphotères comme les N-acyl-aminoacides tels que les N-alkyl-aminoacétates et le cocoamphodiacetate disodique, et les oxydes d'amines tels que l'oxyde de stéaramine, ou encore des tensioactifs siliconés comme les diméthicone copolyols phosphates tels que celui vendu sous la dénomination PECOSIL PS 100® par la société PHOENIX CHEMICAL.

Selon un mode de réalisation, les compositions selon l'invention comprennent comme système émulsionnant l'association suivante :

- d'au moins un agent tensioactif phosphate d'alkyle en $C_{10}$-$C_{30}$, et
- d'au moins un éther d'alcool gras en $C_8$-$C_{24}$ et de polyéthylène glycol, ledit éther comprenant de 1 à 19 motifs oxyéthylène et présentant une HLB< 8 à 25°C.

Selon un mode de réalisation, ledit système émulsionnant peut comprendre en outre au moins un éther d'alcool gras en $C_8$-$C_{24}$ et de polyéthylène glycol, ledit éther comprenant de 20 à 1000 motifs oxyéthylène et de HLB > 8 à 25°C.

Selon un mode de réalisation avantageux, les compositions cosmétiques selon la présente invention comprennent moins de 1 %, de préférence moins de 0,5 % en poids par rapport au poids total de la composition, de triéthanolamine et mieux, est exempte de triéthanolamine.

Selon une variante avantageuse, les compositions cosmétiques selon l'invention comprennent moins de 1 % en poids, de préférence moins de 0,5 % en poids, par rapport au poids total de la composition, de stéarate de triéthanolamine, et mieux, est exempte de stéarate de triéthanolamine.

Les compositions selon l'invention peuvent aussi contenir au moins un polymère filmogène hydrophile ou lipophile.

Par "polymère filmogène" au sens de la présente invention, on entend un polymère apte à former à lui seul ou en présence d'un agent auxiliaire de filmification, un film macroscopiquement continu et adhérant sur les cils, et de préférence un film cohésif, et mieux encore un film dont la cohésion et les propriétés mécaniques sont telles que ledit film peut être isolable et manipulable isolément, par exemple lorsque ledit film est réalisé par coulage sur une surface anti-adhérente comme une surface téflonée ou siliconée.

De façon générale, lorsqu'il(s) est ou sont présent(s), la teneur en polymère(s) filmogène(s) dans les compositions selon l'invention va de 0,1 à 40 %, de préférence de 0,5 à 30 % et mieux de 1 à 20 % en poids, par rapport au poids total de la composition.

Le polymère filmogène hydrophile peut être un polymère hydrosoluble ou se présenter en dispersion dans un milieu aqueux.

Parmi les polymères filmogènes utilisables dans la composition de la présente invention, on peut citer les polymères synthétiques, de type radicalaire ou de type polycondensat, les polymères d'origine naturelle, et leurs mélanges.

Comme exemples de polymères filmogènes hydrosolubles, on peut citer :

les protéines comme les protéines d'origine végétale telles que les protéines de blé et les protéines de soja ; les protéines d'origine animale telles que les kératines, par exemples les hydrolysats de kératine et les kératines sulfoniques ;
les polymères de cellulose tels que l'hydroxyéthylcellulose, l'hydroxypropylcellulose, la méthylcellulose, l'éthylhydroxyéthyl-cellulose, la carboxyméthylcellulose, ainsi que les dérivés quaternisés de la cellulose ;
les polymères ou copolymères acryliques, tels que les polyacrylates ou les polyméthacrylates ;
les polymères vinyliques, comme les polyvinylpyrrolidones, les copolymères de l'éther méthylvinylique et de l'anhydride malique, les copolymères de l'acétate de vinyle et de l'acide crotonique, les copolymères de vinylpyrrolidone et d'acétate de vinyle ; les copolymères de vinylpyrrolidone et de caprolactame ; les poly(alcool vinylique) ;
les polymères de chitine ou de chitosane anioniques, cationiques, amphotères ou non-ioniques ;
les gommes arabiques, la gomme de guar, les dérivés du xanthane, la gomme de karaya ;
les alginates et les carraghénanes ;
les glycoaminoglycanes, l'acide hyaluronique et ses dérivés ;
la résine shellac, la gomme de sandaraque, les dammars, les élémis, les copals ;
l'acide désoxyribonucléïque ;
les muccopolysaccharides tels les chondroïtines sulfate,
et leurs mélanges.

Le polymère filmogène peut être également présent dans la composition sous la forme de particules en dispersion dans une phase aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO, Syntran 5760® par la société Interpolymer Allianz, Opt® par la société Rohm and Haas ; les dispersions aqueuses de polyuréthane vendues sous les dénominations Neorez R-981® et Neorez R-974® par la société AVECIA-NEORESINS, Avalure UR-405®, Avalure UR-410®, Avalure UR-425®, Avalure UR-450®, Sancure 875®, Avalure UR-445® et Sancure 2060® par la société NOVEON, Impranil 85® par la société BAYER, Aquamere H-1511® par la société HYDROMER ; les sulfopolyesters vendus sous le nom de marque Eastman AQ® par la société Eastman Chemical Products ; les dispersions vinyliques comme le Mexomère PAM® ; les dispersions aqueuses de poly(acétate de vinyle) comme le "Vinybran®" de la société Nisshin Chemical ou celles commercialisées par la société UNION CARBIDE ; les dispersions aqueuses de terpolymère vinylpyrrolidone/diméthylaminopropyl méthacrylamide/chlorure de lauryldiméthylpropylméthacrylamidoammonium telles que le Styleze W-d'ISP ; les dispersions aqueuses de polymères hybrides polyuréthane/polyacryliques telles que celles commercialisées sous les références "Hybridur®" par la société AIR PRODUCTS ou "Duromer®" de NATIONAL STARCH ; les dispersions type noyau/enveloppe (ou type « core/shell »), par exemple celles commercialisées par la société ATOFINA sous la référence Kynar (noyau fluoré/enveloppe acrylique) ou encore celles décrites dans le document US 5 188 899 (noyau silice/ enveloppe silicone) ; et leurs mélanges.

Le polymère lipophile peut être en solution ou en dispersion dans une phase solvant non aqueuse.

Comme exemples de dispersions non aqueuses de polymère filmogène, on peut citer les dispersions décrites, par exemple, dans le document EP 749 746 et notamment les particules de polymères acryliques, stabilisées en surface par un stabilisant, en dispersion dans une phase grasse (par exemple l'isododécane) comme le Mexomère PAP® de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

Les compositions selon la présente demande peuvent aussi contenir au moins un gélifiant hydrophile. Ils peuvent être notamment choisis parmi :

- les homo- ou copolymères d'acides acrylique ou méthacrylique ou leurs sels et leurs esters, et en particulier les produits vendus sous les dénominations VERSICOL F® ou VERSICOL K® par la société ALLIED COLLOID, UTRAHOLD 8® par la société CIBA-GEIGY, les poly(acide acrylique) de type SYNTHALEN K,
- les copolymères d'acide acrylique et d'acrylamide vendus sous la forme de leur sel de sodium, sous les dénominations RETEN® par la société HERCULES, les sels de sodium d'acides polyhydroxycarboxyliques vendus sous la dénomination HYDAGEN F® par la société HENKEL,
- les copolymères acide acrylique/acrylates d'alkyle de type PEMULEN,
- les polymères d'acide 2-acrylamido-2-méthylpropane-sulfonique (ou AMPS) neutralisés partiellement à l'ammoniaque et hautement réticulés, tels que celui commercialisé par la société CLARIANT,
- les copolymères AMPS/acrylamide de type SEPIGEL® ou SIMULGEL® commercialisés par la société SEPPIC, et
- les copolymères AMPS/méthacrylates d'alkyle polyoxyéthylénés, réticulés ou non, et leurs mélanges.
- les polyuréthanes associatifs tels que le polymère C16-OE 120-C16 de la société SERVO DELDEN (commercialisé sous le nom SER AD FX1100, molécule à fonction uréthanne et masse moléculaire moyenne en poids de 1300), OE étant un motif oxyéthyléné, le Rhéolate 205 à fonction urée vendu par la société RHEOX ou encore le Rhéolate 208 ou 204 (ces polymères étant vendus sous forme pure) ou le DW 1206B de chez RHOM & HAAS à chaîne alkyle en $C_{20}$ et à liaison uréthane, vendu à 20 % en matière active dans l'eau. On peut aussi utiliser des solutions ou dispersions de ces polyuréthanes associatifs notamment dans l'eau ou en milieu hydroalcoolique. A titre d'exemple, de tels polymères on peut citer, le SER AD FX1010, le SER AD FX1035 et le SER AD 1070 de la société SERVO DELDEN, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCHERS,
- et leurs mélanges.

Certains polymères filmogènes hydrosolubles cités plus haut peuvent également jouer le rôle de gélifiant hydrosoluble.

Lorsqu'il(s) est (sont) présent(s) dans la composition selon l'invention, les gélifiants hydrophiles peuvent être présents dans les compositions selon l'invention en une teneur allant de 0,05 à 40 % en poids par rapport au poids total de la composition, de préférence de 0,1 à 20 % et mieux de 0,5 à 15 % en poids.

Les compositions conformes à l'invention peuvent également comprendre au moins une matière colorante comme les matières pulvérulentes, les colorants liposolubles et les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium ou aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine et le rocou.

Ces matières colorantes peuvent être présentes en une teneur allant de 0,01 à 30 % en poids par rapport au poids total de la composition.

Les compositions conformes à l'invention peuvent également comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon® commercialisé sous la dénomination Orgasol® par la société Atochem, de poly-β-alanine et de polyéthylène, les poudres de polymères de tétrafluoroéthylène comme le Téflon®, la lauroyl-lysine, l'amidon, le nitrure de bore, les microsphères creuses polymériques expansées telles que celles de poly(chlorure de vinylidène/acrylonitrile) comme celles commercialisées sous la dénomination d'Expancel® par la société Nobel Industrie, les poudres acryliques telles que celles commercialisées sous la dénomination Polytrap® par la société Dow Corning, les particules de poly(méthacrylate de méthyle) et les microbilles de résine de silicone (Tospearls® de Toshiba, par exemple), le carbonate de calcium précipité, le carbonate et l'hydrocarbonate de magnésium, l'hydroxyapatite, les microsphères de silice creuses (Silica Beads® de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc et le myristate de magnésium.

On peut également utiliser un composé susceptible de gonfler à la chaleur et notamment des particules thermoexpansibles telles que les microsphères non expansées de copolymère chlorure de vinylidène/acrylonitrile/méthacrylate de méthyle ou d'homopolymère d'acrylonitrile comme, par exemple, celles commercialisées respectivement sous les références Expancel® 820 DU 40 et Expancel® 007WU par la Société AKZO NOBEL.

Les charges peuvent représenter de 0,1 à 25 %, en particulier de 0,2 à 20 % en poids par rapport au poids total de la composition.

Les compositions conformes à l'invention peuvent également comprendre au moins une fibre qui permet une amélioration de l'effet allongeant.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées, par exemple, tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 μm à 10 mm, de préférence de 0,1 mm à 5 mm, et plus particulièrement de 0,3 mm à 3,5 mm. Leur section peut être comprise dans un cercle de diamètre allant de 2 nm à 500 μm, en particulier allant de 100 nm à 100 μm et plus particulièrement de 1 μm à 50 μm. Le poids ou titre des fibres est souvent donné en denier ou décitex et représente le poids en gramme pour 9 km de fil. Les fibres utilisables dans l'invention peuvent en particulier avoir un titre choisi dans la gamme allant de 0,15 à 30 deniers et notamment de 0,18 à 18 deniers.

Les fibres utilisables dans la composition de l'invention peuvent être choisies parmi les fibres rigides ou non rigides. Elles peuvent être d'origine synthétique ou naturelle, minérales ou organiques.

Par ailleurs, les fibres peuvent être traitées ou non en surface, enrobées ou non, colorées ou non colorées.

A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénominations KERMEL®, KERMEL TECH® par la société RHODIA, ou de poly-(p-phénylène-téréphtalamide) (ou d'aramide) notamment vendues sous la dénomination Kevlar ® par la société DUPONT DE NEMOURS.

Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

Les compositions conformes à l'invention peuvent également comprendre en outre au moins un actif cosmétique.

Comme actifs cosmétiques pouvant être utilisés dans les compositions conformes à l'invention, on peut citer notamment les antioxydants, les conservateurs, les parfums, les neutralisants, les émollients, les épaississants, les agents de coalescence, les plastifiants, les hydratants, les vitamines et les filtres en particulier solaires, et leurs mélanges.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

De préférence la composition selon l'invention est non rincée.

La composition selon l'invention peut être conditionnée dans un récipient délimitant au moins un compartiment qui comprend ladite composition, ledit récipient étant fermé par un élément de fermeture.

Le récipient est de préférence associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits, par exemple, dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture.

L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal ou alliage.

Le récipient est de préférence équipé d'un essoreur disposé au voisinage de l'ouverture du récipient. Un tel essoreur permet d'essuyer l'applicateur et éventuellement, la tige dont il peut être solidaire. Un tel essoreur est décrit, par exemple, dans le brevet FR 2 792 618.

Les compositions conformes à l'invention peuvent être utilisées pour le maquillage des fibres kératiniques, en particulier des cils, par exemple comme mascaras.

Un autre objet de l'invention est un procédé de traitement cosmétique des fibres kératiniques, telles que les cils, et plus particulièrement de maquillage ou de revêtement desdites fibres, qui consiste à appliquer une quantité efficace d'une composition telle que décrite ci-dessus, sur lesdites fibres.

Les exemples suivants illustrent la présente invention. Les quantités indiquées ci-après sont exprimées en pourcentage en poids par rapport au poids total de la composition.

EXEMPLES

Exemple 1

On a préparé un mascara à partir des ingrédients indiqués dans le tableau ci-dessous.

| Ingrédients | |
|---|---|
| Cire de candelilla | 16 |
| Cire de carnauba | 4 |
| Huile de jojoba (Simmondsia Chinensis) isomérisée trans de Tf 45 °C [1] | 5 |
| Oxyde de fer noir | 7 |
| Gomme arabique | 3,4 |
| Hydroxyéthylcellulose | 0,9 |
| Potassium cetyl phosphate [2] | 2,5 |
| Alcool stéarylique oxyéthyléné (20 motifs d'oxyde d'éthylène ou (OE)) [3] | 5,2 |
| Alcool stéarylique oxyéthyléné (2 motifs d'OE) [4] | 2,4 |
| Alcool cétylique pur | 2,3 |
| Conservateurs | qs |

(suite)

| Ingrédients | |
| --- | --- |
| Eau | qsp 100 |

[1] vendue sous la dénomination commerciale Iso-Jojoba-50® par la société Desert Whale
[2] vendu sous la dénomination commerciale Amphisol K par la société Givaudan
[3] vendu sous la dénomination commerciale BRIJ 78 par la société UNIQEMA
[4] vendu sous la dénomination commerciale BRIJ 72 par la société UNIQEMA

Pour cette composition de mascara, on a une teneur en cires dures de 22,3 % en poids, une teneur en composés pâteux de 5 % en poids et un rapport pondéral cires dures/(composés pâteux) de 4,46.

On a mélangé les ingrédients de la phase grasse, et on l'a chauffé à 98 °C. On a ajouté ensuite la phase aqueuse préalablement chauffée à 93 °C sous agitation.

On a obtenu une texture particulièrement adaptée à l'application par brosse, ce qui a conduit à un maquillage épais des cils.

Exemple 2

On a préparé un mascara à partir des ingrédients indiqués dans le tableau ci-dessous.

| Ingrédients | |
| --- | --- |
| Cire de candelilla | 7,86 |
| Cire de carnauba | 3,21 |
| Huile de jojoba (Simmondsia Chinensis) isomérisée trans de Tf 45 °C [1] | 4,07 |
| Beurre de karité [2] | 5 |
| Oxyde de fer noir | 7,14 |
| Gomme arabique | 3,4 |
| Hydroxyéthylcellulose | 0,9 |
| Hexadécyl-hydrogenophosphate de potassium [3] | 2,18 |
| Alcool stéarylique oxyéthyléné (20 motifs d'OE) [4] | 4,44 |
| Alcool stéarylique oxyéthyléné (2 motifs d'OE) [5] | 2,1 |
| Alcool cétylique pur | 2 |
| Simethicone | 0,13 |
| Conservateurs | qs |
| Eau | qsp 100 |

[1] vendue sous la dénomination commerciale Iso-Jojoba-50® par la société Desert Whale
[2] vendu sous la dénomination commerciale Lipex Sheasoft par la société AARHUSKARLSHAMN
[3] vendu sous la dénomination commerciale Arlatone MAP 160K par la société UNIQEMA
[4] vendu sous la dénomination commerciale BRIJ 78 par la société UNIQEMA
[5] vendu sous la dénomination commerciale BRIJ 72 par la société UNIQEMA

Pour cette composition de mascara, on a une teneur en cires dures de 13,07 % en poids, une teneur en composés pâteux de 9,07 % en poids et un rapport pondéral cires dures/(composés pâteux) de 1,44.

Exemple 3

On a préparé un mascara à partir des ingrédients indiqués dans le tableau ci-dessous.

| Ingrédients | |
|---|---|
| Cire de candelilla | 7,86 |
| Cire de carnauba | 3,21 |
| Huile de jojoba (Simmondsia Chinensis) isomérisée trans de Tf 45 °C [1] | 4,07 |
| Cire d'orange [2] | 5 |
| Oxyde de fer noir | 7,14 |
| Gomme arabique | 3,4 |
| Hydroxyéthylcellulose | 0,9 |
| Hexadécyl-hydrogenophosphate de potassium [3] | 2,18 |
| Alcool stéarylique oxyéthyléné (20 motifs d'OE) [4] | 4,44 |
| Alcool stéarylique oxyéthyléné (2 motifs d'OE) [5] | 2,1 |
| Alcool cétylique pur | 2 |
| Simethicone | 0,13 |
| Conservateurs | qs |
| Eau | qsp 100 |
| [1] vendue sous la dénomination commerciale Iso-Jojoba-50® par la société Desert Whale<br>[2] vendu sous la dénomination commerciale Orange Peel Wax par la société Koster Keunen<br>[3] vendu sous la dénomination commerciale Arlatone MAP 160K par la société UNIQEMA<br>[4] vendu sous la dénomination commerciale BRIJ 78 par la société UNIQEMA<br>[5] vendu sous la dénomination commerciale BRIJ 72 par la société UNIQEMA | |

Pour cette composition de mascara, on a une teneur en cires dures de 13,07 % en poids, une teneur en composés pâteux de 9,07 % en poids et un rapport pondéral cires dures/(composés pâteux) de 1,44.

**Revendications**

1. Composition cosmétique comprenant une phase aqueuse, au moins 10 % en poids, par rapport au poids total de la composition, d'au moins une cire dure, et au moins un composé pâteux.

2. Composition selon la revendication 1, **caractérisée en ce qu'**elle comprend au moins une huile.

3. Composition selon la revendication 1 ou 2, **caractérisée en ce que** le rapport pondéral cire(s) dure(s)/(composé (s) pâteux+éventuelle(s) huile(s)) est compris dans l'intervalle allant de 2 à 8.

4. Composition cosmétique comprenant une phase aqueuse, au moins une cire dure, au moins un composé pâteux, et éventuellement au moins un huile, le rapport pondéral cire(s) dure(s)/(composé(s) pâteux+éventuelle(s) huile(s)) étant compris dans l'intervalle allant de 2 à 8.

5. Composition selon l'une quelconque des revendications 2 à 4, **caractérisée en ce que** la quantité totale de composé (s) pâteux et d'huile(s) est supérieure à 1 % en poids, de préférence allant de 1 à 15 % en poids par rapport au poids total de la composition.

6. Composition selon l'une quelconque des revendications précédentes, **caractérisée en ce que** la ou les cires dures présentent une dureté à 20 °C, supérieure à 5 MPa, de préférence allant de 5 à 30 MPa.

7. Composition selon l'une quelconque des revendications précédentes, **caractérisée en ce que** la ou les cires dures sont choisies parmi la cire de Carnauba, la cire de candelilla, la cire BIS-PEG-12 DIMETHICONE CANDELILLATE, la cire de Jojoba hydrogénée, l'huile de palme hydrogénée, la cire de son de riz, la cire de Sumac, les cires de cérésine, la cire de laurier, la cire d'insecte Chinois, la cire de Shellac, l'huile d'olive hydrogénée, les cires obtenues par hydrogénation d'huile d'olive estérifiée avec des alcool gras à chaîne en C12 à C18, les cires obtenues par hydrogénation d'huile de ricin estérifiée avec l'alcool cétylique ou béhénique, la cire de Cameline hydrogénée, la cire d'Ouricury, la cire de Montan, les cires d'ozokerite, les cires microcristallines, les triglycérides d'acides laurique, palmitique, cétylique et stéarique, les cires de polyméthylène, les cires de polyéthylène, les cires d'alcool-polyéthylène, le copolymère éthylène/acide acrylique 95/5, l'hydroxystéarate d'hydroxyoctacosanyle, le stéarate d'octacosanyle, le stéarate de stéaryle, le distéarate de pentaérythritol, le mélange d'adipate de dibéhényle, d'adipate de dioctadécyle et d'adipate de dieicosanyle, le mélange d'adipate de dilauryle et d'adipate de ditétradécyle, le mélange de sébaçate de dioctadécyle, de sébaçate didocosyle et de sébaçate dieicosyle, le mélange d'octadécanedioate de dioctadécyle, d'octanedioate de didocosyle et d'octanedioate de dieicosyle, le tétrastéarate de pentaérythrityle, le stéarate de tétracontanyle, le benzoate de stéaryle, le fumarate de béhényle, le tétrastéarate de di-(triméthylol-1,1,1-propane), le distéarate de didotriacontanyle, le montanate de polyéthylène glycol à 4 motifs oxyéthylène, le disalycilate d'hexanediol, l'hexastéarate de dipentaérythrytol, le tétrabéhénate de ditriméthylolpropane, les esters de Jojoba, les mélanges d'acide carboxylique linéaire (C20-40)/hydrocarbures saturés, la cire synthétique de type Fischer-Tropsch, l'alcool cétylique, l'alcool stéarique, l'alcool béhénique, le carbonate de dioctadécyle et le polybéhénate de saccharose.

8. Composition selon la revendication 7, **caractérisée en ce que** la ou les cires dures sont choisies parmi la cire de carnauba, la cire de candellila, la cire de jojoba hydrogénée, la cire de sumac, les cires obtenues par hydrogénation d'huile d'olive estérifiée avec les alcool gras à chaîne en C12 à C18, la cire de son de riz, les alcools cétylique, stéarylique et béhéniques, la cire de laurier et la cire d'Ouricury.

9. Composition selon l'une quelconque des revendications précédentes, **caractérisée en ce qu'**elle comprend au moins 12 % en poids, de préférence de 12 % à 30 % en poids par rapport au poids total de la composition, d'au moins une cire dure.

10. Composition selon l'une quelconque des revendications précédentes, **caractérisée en ce que** le ou les composés pâteux sont des composés gras lipophiles à changement d'état solide/liquide réversible et comportant à la température de 23°C une fraction liquide et une fraction solide.

11. Composition selon l'une quelconque des revendications précédentes, **caractérisée en ce que** le ou les composés pâteux sont choisis parmi des composés synthétiques et des composés d'origine végétale.

12. Composition selon la revendication 11, **caractérisée en ce que** la ou les composés pâteux sont choisis parmi :

- la lanoline et ses dérivés,
- les composés siliconés polymères ou non-polymères,
- les composés fluorés polymères ou non-polymères,
- les polymères vinyliques, notamment

  • les homopolymères d'oléfines,
  • les copolymères d'oléfines,
  • les homopolymères et copolymères de diènes hydrogénés,
  • les oligomères linéaires ou ramifiés, homo ou copolymères de (méth)acrylates d'alkyle ayant de préférence un groupement alkyle en $C_8$-$C_{30}$
  • les oligomères homo et copolymères d'esters vinyliques ayant des groupements alkyles en $C_8$-$C_{30}$,
  • les oligomères homo et copolymères de vinyléthers ayant des groupements alkyles en $C_8$-$C_{30}$,

- les polyéthers liposolubles résultant de la polyéthérification entre un ou plusieurs diols en $C_2$-$C_{100}$, de préférence en $C_2$-$C_{50}$,
- les esters et les polyesters,
- et leurs mélanges.

**13.** Composition selon la revendication 11, le composé pâteux est choisi parmi l'huile de jojoba partiellement hydrogénée isomérisée trans, la cire d'orange, le beurre de karité, l'huile d'olive partiellement hydrogénée, le beurre de cacao et l'huile de mangue.

**14.** Composition selon l'une quelconque des revendications précédentes, **caractérisée en ce que** le ou les composés pâteux sont présents en une quantité supérieure à 1 % en poids, de préférence allant de 1 à 15 % en poids par rapport au poids total de la composition.

**15.** Composition selon l'une quelconque des revendications 2 à 14, **caractérisée en ce que** la ou les huiles sont volatiles ou non volatiles, et sont choisies parmi des huiles hydrocarbonées, des huiles siliconées, des huiles fluorées et leurs mélanges.

**16.** Composition selon l'une quelconque des revendications précédentes, **caractérisée en ce qu'**elle présente une teneur en matière sèche supérieure ou égale à 40% en poids, de préférence supérieure ou égale à 45% en poids par rapport au poids total de la composition.

**17.** Composition selon l'une quelconque des revendications précédentes, **caractérisée en ce que** la phase aqueuse comprend de l'eau et éventuellement au moins un solvant hydrosoluble.

**18.** Composition selon l'une quelconque des revendications précédentes, **caractérisée en ce qu'**elle comprend au moins une cire additionnelle présentant une dureté inférieure ou égale à 5 MPa à 20 °C.

**19.** Composition selon l'une quelconque des revendications précédentes, **caractérisée en ce qu'**elle comprend un ou plusieurs agents tensioactifs émulsionnants.

**20.** Procédé de maquillage des fibres kératiniques, telles que les cils, qui consiste à appliquer une quantité efficace d'une composition selon l'une quelconque des revendications 1 à 19, sur lesdites fibres.

## RÉFÉRENCES CITÉES DANS LA DESCRIPTION

**Documents brevets cités dans la description**

- EP 847752 A
- US 5188899 A
- EP 749746 A
- WO 04055081 A
- US 4887622 A
- FR 2796529
- FR 2761959
- FR 2792618

**Littérature non-brevet citée dans la description**

- **VAN DE HULST, H.C.** Light Scattering by Small Particles. Wiley, 1957
- **GRIFFIN.** *J. Soc. Cosm. Chem.,* 1954, vol. 5, 249-256
- **KIRK-OTHMER.** Encyclopedia of Chemical Technology. WILEY, 1979, vol. 22, 333-432347-377